(19) Europäisches Patentamt
European Patent Office
Office européen des brevets

(11) **EP 1 807 458 B1**

(12) **EUROPEAN PATENT SPECIFICATION**

| | |
|---|---|
| (45) Date of publication and mention of the grant of the patent:<br>**07.05.2014  Bulletin 2014/19** | (51) Int Cl.:<br>***C08F 265/06*** *(2006.01)*   ***A61K 6/083*** *(2006.01)*<br>***C08L 51/00*** *(2006.01)* |
| (21) Application number: **05800115.7** | (86) International application number:<br>**PCT/GB2005/004262** |
| (22) Date of filing: **03.11.2005** | (87) International publication number:<br>**WO 2006/048661 (11.05.2006 Gazette 2006/19)** |

(54) **PRODUCTION OF ACRYLATE-BASED SOLID**

HERSTELLUNG EINES ACRYLATFESTKÖRPERS

PRODUCTION DE MATIERE SOLIDE A BASE D'ACRYLATE

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR** | (74) Representative: **Jappy, John William Graham**<br>**Gill Jennings & Every LLP**<br>**The Broadgate Tower**<br>**20 Primrose Street**<br>**London EC2A 2ES (GB)** |
| (30) Priority: **03.11.2004  GB 0424353**<br>**16.12.2004  GB 0427576** | (56) References cited:<br>**EP-A- 0 786 479    EP-A- 0 894 828**<br>**GB-A- 569 684    GB-A- 1 037 904**<br>**US-A- 3 637 559    US-A- 3 974 104**<br>**US-A- 4 490 497    US-A- 5 747 553**<br>**US-A- 5 985 998    US-A- 6 103 779** |
| (43) Date of publication of application:<br>**18.07.2007  Bulletin 2007/29** | |
| (73) Proprietor: **Makevale Group Ltd.**<br>**Hertfordshire SG12 9QP (GB)** | Remarks:<br>The file contains technical information submitted after the application was filed and not included in this specification |
| (72) Inventor: **AHIR, Vallabhbhai, Bhanabhai**<br>**Makevale Group Ltd.**<br>**Hertfordshire SG12 9QP (GB)** | |

**Description**

Field of the Invention

This invention relates to the production of compositions that are useful for producing cast/moulded acrylic products.

Background of the Invention

Acrylate-based plastics, commonly known as acrylics, are amorphous and or crystalline thermoplastics with a high strength to weight ratio, high stability and good resistance to environmental elements. The most common example is polymethylmethacrylate, which is transparent and often used as a glass replacement. Acrylate-based plastics are also used extensively in the casting and moulding industry, where plastics of any desired shape can be produced, for example products such as dentures, toys, household goods and automotive parts amongst others.

The standard technique used to produce an acrylate-based plastic involves dispersing an acrylate polymer/copolymer in an acrylic monomer. For example, three parts of polymethylmethacrylate (PMMA) may be combined with two parts of methylmethacrylate monomer (MMA). This mixture is then intermittently stirred for up to 20 minutes to achieve a homogenous mixture. During this time, the polymer will absorb the monomer to give a very viscous fluid mixture of polymer in monomer. If allowed to continue, a gel is formed, commonly known as a "dough". At a suitable point in this process, for example when the fluid mixture just flows, it is poured into a mould. This is then left to stand at room temperature for up to 4 hours. The mould is then placed inside a pressure vessel and pressurised to between 80 and 150 psig and placed in an oven at 40-85ºC for a minimum of 6 hours. The entire curing cycle therefore can takes up to 20 hours to complete.

Furthermore, castings at present may require curing in several separate layers to ensure complete curing, particularly if the casting is large or deep. This further lengthens the time and complexity of the procedure.

In many applications, including the production of dentures, the polymer/monomer mixture is prepared by hand, with a new mixture prepared for each separate casting. This can lead to several possible problems. Firstly, the acrylate chemicals are well known as possibly causing dermatitis/sensitisation in susceptible persons on contact with the skin. Acrylates can also have an unpleasant odour, and high flammability. At higher atmospheric levels they can also cause irritation to the eyes, nose and throat. It is recognised that containment of these chemicals, such as to reduce contact with the skin and minimise vapours entering the air, is preferred over the use of personal protective equipment such as gloves. However, the current methods of producing acrylate based solids limit the level of containment possible. Furthermore, the preparation of a new batch of polymer/monomer mix for each casting inevitably leads to excessive wastage.

There is, therefore, the need for a process to produce acrylate-based products that, not only, reduce production time but which can possibly be automated, minimise wastage and, in particular, dramatically improve environmental issues.

Summary of the Invention

The present invention is based on the surprising discovery that a two-step process involving the dispersion of a small quantity of a polyacrylate polymer/copolymer into an acrylic monomer prior to adding a further amount of an acrylate polymer/copolymer, results in a considerable reduction in the overall time taken to produce the products. The method of producing acrylate-based plastics according to the invention is also capable of automation, reducing wastage and allowing for a tidier and cleaner production process.

According to a first aspect of the invention, a method for producing an acrylate-based solid comprises the steps of:

(i) dispersing an amount of an alkyl or aryl acrylate polymer/copolymer in a solvent comprising at least one acrylic monomer;
(ii) dispersing in the fluid mixture formed by step (i) a further quantity of an alkyl or aryl acrylate polymer/copolymer; and
(iii) curing the material resulting from step (ii) to form a solid,

wherein the amount of alkyl or aryl acrylate polymer/copolymer dispersed in step (i) is less than 20% w/v and wherein the alkyl or aryl acrylate polymer/copolymer of step (i) contains less than 0.1% w/w of a free radical polymerisation initiator, wherein the polymerisation initiator is a peroxide or a percarbonate.

According to a second aspect of the invention, a composition comprises less than 20% w/v of an alkyl or aryl acrylate polymer/copolymer that is substantially free of free radicals dispersed in a solvent comprising at least one acrylic monomer. This composition may be mixed with a further amount of an alkyl or aryl acrylate polymer/copolymer, to form a material with a viscosity greater than the composition comprising less than 20% w/v of an acrylate polymer, which is suitable for casting/moulding.

According to a third aspect of the invention, a kit for the production of an acrylate-based solid comprises a composition comprising less than 20% w/v of an alkyl and/or aryl acrylate polymer/copolymer that is substantially free of free radicals, dispersed in a solvent comprising at least one acrylic monomer, and a separate second composition comprising at least one alkyl and/or aryl acrylate polymer/copolymer, wherein the solid is produced by combining the first composition with the second composition and curing the resulting material.

According to a fourth aspect of the invention, a device for the injectable delivery of an acrylate-based composition into a mould comprises a first discrete compartment containing a composition comprising less than 20% w/v of an alkyl and/or aryl acrylate polymer/copolymer that is substantially free of a free radical dispersed in a solvent comprising at least one acrylic monomer, and a second discrete compartment comprising at least one alkyl and/or aryl acrylate polymer/copolymer, the device containing a nozzle for the simultaneous expulsion of the composition and the polymer from the device.

According to a fifth aspect of the invention, a device for the injectable delivery of an acrylate-based composition into a mould comprises a first discrete compartment containing a composition comprising less than 20% w/v of an alkyl and/or aryl acrylate polymer/copolymer that is substantially free of a free radical dispersed in a solvent comprising at least one acrylic monomer, and a second discrete compartment comprising at least one alkyl and/or aryl acrylate polymer/copolymer, the device containing separate apertures/ nozzles for the simultaneous expulsion of the composition and the polymer from the device under controlled conditions whereby they are then mixed prior to injection.

Detailed Description of the Invention

The present invention identifies that an acrylate-based solid with a reduced curing and overall production time can be produced by initially dispersing less than 20% w/v of an alkyl and/or aryl acrylate polymer/copolymer that is substantially free of a free radical polymerisation initiator compound in a solvent comprising an acrylic monomer with a viscosity of 100cP or greater. This forms a viscous fluid mixture "syrup". Combining this fluid mixture with additional alkyl and/or aryl acrylate polymer/copolymer results in a material with a much shortened processing time, usually less than 2 hours. The fluid mixture containing the alkyl and/or aryl acrylate polymer/copolymer that is substantially free of a free-radical polymerisation initiator compound is stable for a minimum of three months at temperatures below 25$\underline{o}$C. This is in contrast to many of the prior art mixtures which remain stable for only a few hours.

Without wishing to be bound by theory, it is thought that forming the fluid mixture (comprising less than 20% w/v alkyl and/or aryl acrylate polymer/copolymer that is substantially free of a free-radical polymerisation initiator compound in an acrylic monomer solvent) increases the speed of the reaction that occurs when a further amount of polymer is added. The overall time taken to produce acrylate solids is therefore reduced.

As used herein, the term "acrylate" refers to acrylic acid or any ester of acrylic acid, with the formula $CH_2C(R1)COOR2$, wherein R1 and R2 can be an organic group, or Hydrogen. R1 and R2 can be the same or different. Preferably, the R2 group is an alkyl or aryl group. R1 is preferably Hydrogen or an alkyl or aryl group. Commonly used groups are methyl, ethyl, butyl, lauryl and stearyl, as will be appreciated by one skilled in the art. More preferably, the R groups (R1 and R2) are methyl groups, and the acrylate is therefore methylmethacrylate (MMA).

Acrylate monomers can polymerise to form an acrylate polymer by polymerisation about the vinyl group, as will be appreciated by one skilled in the art. As used herein, the term "acrylate polymer" refers to any molecule comprising two or more acrylate monomers linked covalently via the vinyl groups. The polymer may be a homopolymer and consist of only a single type of monomer, or may be a co-polymer, consisting of at least two different monomer units. As indicated above, a preferred acrylate polymer is (the homopolymer) polymethylmethacrylate (PMMA).

The polymer that is added in an amount up to 20%w/v to form the fluid mixture in the first step (step (i)) of the method according to the invention, is substantially free of a free-radical polymerisation initiator. As used herein, the term "substantially free" refers to a polymer that contains less than 0.1 % w/w of a free radical. Preferably, the polymer that is used in step (i) contains less than 0.08% w/w of a free radical polymerisation initiator. The skilled person will appreciate that a free radical polymerisation initiator may be used in the production of the polymer itself. If there is a residual amount of a free radical compound in the polymer after polymerisation, i.e. greater than 0.1 % w/w, its concentration may be reduced, i.e. to less than 0.1 w/w, by heating the polymer mixture to a maximum temperature of approximately 130°C for between 10 and 60 minutes, for example 45 minutes. This will decompose the free radical compound, resulting in a polymer that is substantially free of a free radical polymerisation initiator.

A method for determining the percentage w/w of a free radical in a sample of an acrylate polymer is given in Example 8. Preferably, the free radical that is determined using this method is benzoyl peroxide.

As used herein, the term "free radical" refers to an agent used to polymerise acrylate monomers. These are often referred to as polymerisation initiators or agents. According to the present invention, the term refers to a peroxide, more preferably an organic peroxide, most preferably benzoyl peroxide. The term also includes percarbonates such as sodium percarbonate. For the avoidance of doubt, a polymer that is added in the first step of the method according to the present invention is substantially free of a polymerisation initiator, the common initiator being benzoyl peroxide.

Acrylate polymers containing any number of monomer units may be used in the current invention. Preferably, the polymer contains a large number of monomer units so that each polymer molecule has an average molecular weight of greater than 100,000, more preferably greater than 400,000 and most preferably between 500,000 and 2,000,000, for example 1,000,000 or 1,500,000.

Suitable polymers include MV650, LP-22, LP10, MV720/721, D-88-S, D-300-S, MV11 and MV12. All of these are commercially available from Makevale Group Limited, Valley House, Marsh Lane, Ware, Hertfordshire, United Kingdom.

The method of the invention relates to the production of acrylate-based solids. As used herein, the term "acrylate-based solid" refers to any solid plastic material formed by the polymerisation of acrylate monomers.

The invention involves dispersing less than 20% w/v of an alkyl or aryl acrylate polymer/copolymer that is substantially free of a free-radical polymerisation initiator in a solvent comprising at least one acrylic monomer, to form a fluid mixture. Preferably, less than 15%, for example 13%, 10% or 5% of the acrylate polymer/copolymer is dispersed. The acrylate polymer may be a homopolymer or a copolymer. Preferably, the acrylate polymer is in the form of a particulate solid, for example in the form of beads, micro beads or micro spheres. More preferably, the polymer is a powder. Polyacrylate powders are well known to one skilled in the art and are commercially available.

The acrylate polymer/copolymer that is substantially free of a free-radical polymerisation initiator is dispersed in a solvent in step (i) of the method, to form a fluid mixture. The solvent contains at least one acrylic monomer. As used herein, the term "acrylic monomer" is well known to one skilled in the art and refers to acrylic acid or any ester of acrylic acid with the formula $CH_2C(R1)COOR2$, wherein R1 and R2 are preferably a hydrogen atom, alkyl or aryl group. Preferred R1 and R2 groups include, but are not limited to methyl, ethyl, butyl, lauryl, stearyl and 2-ethylhexyl groups. Preferred compounds therefore include, but are not limited to methyl acrylate, butyl acrylate, ethyl acrylate, and 2-ethylhexyl acrylate, or derivatives thereof. Preferably, the acrylic monomer is an acrylate monomer or derivative thereof. More preferably, the acrylic monomer is methyl methacrylate (MMA). The solvent may contain a single acrylic monomer or a mixture of different acrylic monomers, for example 2, 3, 4 or more different monomers.

Other agents may also be present in the solvent, including fillers, dyes and other agents that give the resultant plastic desirable characteristics. It should be noted that an acrylate based solid can be produced according to the method of the invention without any (of these) additional agents. Examples of suitable additives include di-isobutyl phthalate (DIBP), di-butyl phthalate (DBP), Alumina Trihydrate (ATH) and ethylene glycol di-methacrylate (EGDM).

The fluid mixture formed by step (i) of the method does not require free radical initiators (often termed polymerisation initiators or agents). As indicated above, the polymer that is added to the solvent in step (i) is substantially free of a free radical polymerisation initiator.

Once 20% w/v or less of the alkyl or aryl acrylate polymer/copolymer that is substantially free of a free radical polymerisation initiator has been dispersed in the acrylic monomer solvent, the fluid mixture formed is viscous. Preferably, the fluid mixture has a viscosity of between 100cP and 2000cP, more preferably 500cP-1800cP, most preferably 1600cP.

This viscous fluid mixture is stable when stored for three months and possibly up to 2 years at ambient temperature and pressure, and may be made in bulk and stored until required. The fluid mixture can be stabilised and stored for three or more years at ambient temperature and pressure.

To form a solid plastic according to the method of the invention a further quantity of an alkyl or aryl acrylate polymer/copolymer is then added to this viscous fluid mixture. A single polymer may be added, or a combination of polymers. The polymer may be different to that used in the first step and may be added up to 75% w/v. Again, this polymer is preferably a particulate solid, such as beads, micro beads, micro spheres or, most preferably, a powder. The polymer that is added in this step (step (ii)) does not have to be substantially free of a free radical polymerisation initiator. In a preferred embodiment, the polymer added in step (ii) contains a free-radical compound, i.e. at an amount greater than 0.1 % w/w, for example greater than 0.2% or 0.3% w/w. Alternatively, a suitable amount of a free radical compound can be added to the fluid mixture formed by step (i) together (simultaneously or sequentially) with the further amount of polymer.

In an alternative embodiment, the method of the invention can utilise a polymer in step (ii) that is substantially free of a free-radical polymerisation initiator, without the need for addition of a free radical. In this embodiment, the entire method of the invention is substantially free of a free radical polymerisation initiator.

Adding the further acrylate polymer/copolymer to the fluid mixture causes the viscosity of the mixture to thicken almost instantly into a pourable or doughed material, with a viscosity greater than the initial dispersion. Preferably, the viscosity of the material produced by this step (step (ii)) is between 100 cP and 2000cP, more preferably between 500cP and 1800cP, most preferably greater than 1600cP.

The terms "pourable" and "doughed" are well known by those skilled in the art to refer to a material that is suitable for pouring into a cast or mould, with a viscosity between 100cP and 2000cP. This material may then be fashioned or worked into any desired shape, preferably by pouring into a cast or mould. Any suitable mould may be used, a preferred mould is a dental mould, used in dentistry, for example to create dentures.

Alternatively, the material may be used to make sheets of glass substitute of any desired shape. Other examples include automotive accessories, decorative paperweights, promotional/advertising products and presentational award plaques.

The prepared material may be poured into a mould or cast. Alternatively, the material may be introduced into a mould or cast by the use of an injection apparatus. Preferably, an injection device delivers the material ready for curing directly into a mould or cast. A suitable injection device comprises a first discrete compartment containing the mixture comprising less than 20% w/v of an alkyl or aryl acrylate polymer/copolymer that is substantially free of a free radical polymerisation initiator dispersed in a solvent comprising at least one acrylic monomer and a second discrete compartment containing at least one alkyl or aryl acrylate polymer/copolymer.

A suitable device may be in the form of a dual chambered "syringe" or "gun" -type apparatus, as will be appreciated by one skilled in the art, and comprises at least two compartments and a nozzle that allows the simultaneous expulsion of the fluid mixture comprising less than 20% w/v of an alkyl or aryl acrylate polymer/copolymer, and the alkyl or aryl acrylate polymer/copolymer. For example, there may be a single nozzle through which both materials are expelled, such that they are forced to mix as they leave the device. Alternatively, each of the two materials may have a separate aperture through which they leave the device, whereby they are then mixed prior to entry into the mould or cast. Preferably, the two materials are expelled from the two compartments by the application of increased pressure upon the compartments, for example by the movement of a piston into each of the compartments. Pressure may be applied to each compartment separately, so that the expulsion of each material can be individually controlled. Pressure may alternatively be applied simultaneously to both compartments, for example by applying equal pressure to each piston. The dimensions of the compartments are so designed as to control the correct component mix.

The two materials do not mix within the separate compartments of the device. Therefore, the injection device can be prepared to contain materials sufficient for several mouldings. Only the required amount needs to be expelled from the device during each use. Due to the stable nature of the materials, the materials can be left in the device, and be re-used when the next application is required. This minimises wastage of materials and encloses the chemicals, reducing the risks associated with acrylates being exposed to the environment.

Once the material is in the desired shape, for example when introduced into a mould, it is cured to form a solid. Methods of curing acrylate-based materials are well known to those skilled in the art. Preferably, curing involves raising the temperature of the material to greater than ambient temperature, preferably between ambient temperature and 100ºC. More preferably, the temperature is raised to at least 50ºC, more preferably 60ºC, even more preferably greater than 80ºC, for example 85ºC. Preferably this heat curing is accompanied by an increase in pressure, preferably greater than 50 psig, more preferably greater than 60 psig and most preferably 80 psig or greater, for example 100psig. Whether heat alone is used, or a combination of heat and pressure, the curing step takes less than 3 hours, more preferably less than 2 hours and most preferably 1 hour or less. Curing may occur in air or in water, or in any other suitable environment apparent to the skilled person.

The material may alternatively be cured at approximately 100ºC, for example in boiling water, with no substantial increase in pressure. In this embodiment, the curing step takes less than 1 hour, preferably less than 30 minutes and most preferably approximately 20 minutes.

The invention is illustrated by reference to the following non-limiting examples.

Example 1

Polymer grade MV650 (polymethylmethacrylate polymer) was incorporated into a fluid mixture comprising between 0.1% and 20% of an alkyl or aryl homopolymer or copolymer dispersed in 100% MMA and a casting prepared. This casting was cured in water for 1.5 hours at 60ºC and 80 psig. Using standard techniques, this casting would take 6 hours to cure.

Example 2

Polymer grade MV650 was mixed with a fluid mixture comprising between 0.1 % and 20% of an alkyl or aryl homopolymer or copolymer dispersed in 100% MMA and a casting prepared. This casting was cured in water for 1 hour at 85ºC and 80 psig.

Example 3

Polymer grades LP-22, MV11 and MV12 (co-polymers of PMMA) were mixed with a fluid mixture comprising between 0.1% and 20% of an alkyl or aryl homopolymer or copolymer dispersed in an acrylic monomer mixture containing at least 90% MMA and the doughed material packed into a dental flask. This was cured for 20 minutes in boiling water at atmospheric pressure.

Example 4

Polymer grades LP-22 and MV11 (co-polymers of PMMA) were mixed with a fluid mixture comprising between 0.1 % and 20% of an alkyl or aryl homopolymer or copolymer dispersed in an acrylic monomer mixture containing at least 90% MMA and the doughed material packed into a dental flask. This was cured in water for 5 minutes at 120°c and 5 bar pressure.

Example 5

Polymer grades MV720 and MV721 (polymethylmethacrylate polymer) was mixed with a dispersion comprising between 0.1 % and 20% of an alkyl or aryl homopolymer or copolymer dispersed in 100% MMA and a casting prepared. This casting was cured for 2 hours at 60°C and 80 psig. Using standard techniques, this casting would normally take 6 hours to cure.

Example 6

Polymer grade MV720 and MV721 (polymethylmethacrylate polymer) was mixed with a fluid mixture comprising between 0.1% and 20% of an alkyl or aryl homopolymer or copolymer dispersed in 100% MMA and a casting prepared. This casting was cured in water for 1.5 hours at 85°C and 80 psig. Using standard techniques, this casting would normally take 6 hours to cure.

Example 7

Polymer grades D-88-S and D-300-S (homo-polymers of PMMA) or LP-10 (a co-polymer of PMMA) were mixed with a fluid mixture comprising between 0.1 % and 20% of an alkyl or aryl homopolymer or copolymer dispersed in an acrylic monomer mixture containing at least 90% MMA but using a powder to liquid ratio of up to 75% and the doughed material packed into a dental flask. These were cured for 20 minutes in boiling water at atmospheric pressure.

Example 8 Determination of the peroxide content in PMMA Polymer by a titration method.

Reagents:

Dichloroethane (LR)
Industrial methylated spirits (IMS)
50% w/w potassium iodide solution (LR)
Glacial acetic acid (ANALAR)
Standardised 0.1M-Sodium thiosulphate (ANALAR)

Method:

1) Weigh accurately about 5 gm of sample into a 500 ml glass stoppered flask.
2) Just "wet" polymer with IMS and then add 50 ml of dichloromethane, swirl immediately to dissolve.
3) When the polymer has completely dissolved add 100 ml of IMS slowly down the side of the flask, then shake vigorously until solution is clear. Add 2 ml of the 50% potassium iodide solution, then 1 ml of glacial acetic acid, shaking the flask during the additions.
4) Seal the flask with a cellulose square and rubber band, allow to stand in the dark for 1 hour. Titrate the liberated iodine with the 0.1N sodium thiosulphate, slowly as the end-point is approached. Near the end-point, shake for 10 seconds between the addition of each drop from the burette.

Carry out a blank determination on the reagents and deduct the titre from the gross titre obtained with the sample.

$$\% \text{ peroxide} = \frac{\text{mls. of 0.1M sod. thio} \times 0.0118 \times 100\%}{\text{Weight of sample}}$$

i.e. % peroxide content (if 5 gm of polymer used = mls of 0.1M sodium thiosulphate x 0.235).

**Claims**

1. A method for producing an acrylate-based solid, comprising the steps of:

   (i) dispersing an amount of an alkyl or aryl acrylate polymer/copolymer in a solvent comprising at least one acrylic monomer;
   (ii) dispersing in the fluid mixture formed by step (i) a further quantity of an alkyl or aryl acrylate polymer/copolymer; and
   (iii) curing the resulting material to form a solid,

   wherein the amount of alkyl or aryl acrylate polymer/copolymer dispersed in step (i) is less than 20% w/v, and wherein the alkyl or aryl acrylate polymer/copolymer of step (i) contains less than 0.1% w/w of a free radical polymerisation initiator that is a peroxide or a percarbonate.

2. A method according to claim 1, wherein the alkyl or aryl acrylate polymer/copolymer of step (i) contains less than 0.08% w/w of a free radical polymerisation initiator.

3. A method according to claim 1 or claim 2, wherein the free radical polymerisation initiator is a peroxide.

4. A method according to any preceding claim, wherein the free radical polymerisation initiator is benzoyl peroxide.

5. A method according to claim 1 or claim 2, wherein the free radical polymerisation initiator is a percarbonate.

6. A method according to any preceding claim, wherein the solvent consists of at least two acrylic monomers.

7. A method according to any preceding claim, wherein the alkyl or aryl acrylate polymer of step (i) and/or step (ii) is polymethyl methacrylate.

8. A method according to any preceding claim, wherein the alkyl or aryl acrylate polymer of step (i) and/or step (ii) has an average molecular weight of at least 100,000.

9. A method according to claim 8, wherein the average molecular weight is between 500,000 and 2,000,000.

10. A method according to any preceding claim, wherein the at least one acrylic monomer is methyl methacrylate.

11. A method according to any preceding claim, wherein the fluid mixture formed in step (i) has a viscosity between 500 and 1800cP.

12. A method according to any preceding claim, wherein the material formed in step (ii) has a viscosity greater than that of the fluid mixture formed in step (i).

13. A method according to any preceding claim, wherein curing is carried out at a temperature of at least 50ºC for less than 2 hours.

14. A method according to claim 13, wherein curing is carried out at a pressure of at least 60 psig.

15. A method according to any preceding claim, wherein the material formed in step (ii) is inserted into a mould prior to curing.

**16.** A method according to claim 15, wherein the mould is a dental mould.

**Patentansprüche**

**1.** Verfahren zur Herstellung eines Acrylat-basierten Feststoffs, umfassend die Schritte von:

(i) Dispergieren einer Menge eines Alkyl- oder Arylacrylat-Polymers/Copolymers in einem Lösungsmittel, umfassend mindestens ein Acrylmonomer;
(ii) Dispergieren einer weiteren Menge eines Alkyl- oder Arylacrylat-Polymers/Copolymers im durch Schritt (i) gebildeten Flüssigkeitsgemisch; und
(iii) Härten des sich ergebenden Materials zum Bilden eines Feststoffs,

wobei die Menge von in Schritt (i) dispergiertem Alkyl- oder Arylacrylat-Polymer/Copolymer weniger als 20 % (w/v) beträgt und wobei das Alkyl- oder Arylacrylat-Polymer/Copolymer von Schritt (i) weniger als 0,1% (w/w) eines Radikalkettenpolymerisationsinitiators, das heißt eines Peroxids oder eines Percarbonats, enthält.

**2.** Verfahren nach Anspruch 1, wobei das Alkyl- oder Arylacrylat-Polymer/Copolymer von Schritt (i) weniger als 0,08 % (w/w) eines Radikalkettenpolymerisationsinitiators enthält.

**3.** Verfahren nach Anspruch 1 oder Anspruch 2, wobei der Radikalkettenpolymerisationsinitiator ein Peroxid ist.

**4.** Verfahren nach einem der vorangehenden Ansprüche, wobei der Radikalkettenpolymerisationsinitiator Benzoylperoxid ist.

**5.** Verfahren nach Anspruch 1 oder Anspruch 2, wobei der Radikalkettenpolymerisationsinitiator ein Percarbonat ist.

**6.** Verfahren nach einem der vorangehenden Ansprüche, wobei das Lösungsmittel aus mindestens zwei Acrylmonomeren besteht.

**7.** Verfahren nach einem der vorangehenden Ansprüche, wobei das Alkyl- oder Arylacrylat-Polymer von Schritt (i) und/oder Schritt (ii) Polymethylmethacrylat ist.

**8.** Verfahren nach einem der vorangehenden Ansprüche, wobei das Alkyl- oder Arylacrylat-Polymer von Schritt (i) und/oder Schritt (ii) ein durchschnittliches Molekulargewicht von mindestens 100.000 aufweist.

**9.** Verfahren nach Anspruch 8, wobei das durchschnittliche Molekulargewicht zwischen 500.000 und 2.000.000 beträgt.

**10.** Verfahren nach einem der vorangehenden Ansprüche, wobei das mindestens eine Acrylmonomer Methylmethacrylat ist.

**11.** Verfahren nach einem der vorangehenden Ansprüche, wobei das in Schritt (i) gebildete Flüssigkeitsgemisch eine Viskosität zwischen 500 und 1800 cP aufweist.

**12.** Verfahren nach einem der vorangehenden Ansprüche, wobei das in Schritt (ii) gebildete Material eine Viskosität größer als die des in Schritt (i) gebildeten Flüssigkeitsgemischs aufweist.

**13.** Verfahren nach einem der vorangehenden Ansprüche, wobei das Härten bei einer Temperatur von mindestens 50 °C für weniger als 2 Stunden durchgeführt wird.

**14.** Verfahren nach Anspruch 13, wobei das Härten bei einem Druck von mindestens 60 psig durchgeführt wird.

**15.** Verfahren nach einem der vorangehenden Ansprüche, wobei das in Schritt (ii) gebildete Material (ii) vor dem Härten in eine Form eingebracht wird.

**16.** Verfahren nach Anspruch 15, wobei die Form eine Zahnabdruckform ist.

**Revendications**

1. Procédé de production de matière solide à base d'acrylate, comprenant les étapes suivantes :

> (i) dispersion d'une quantité de polymère/copolymère d'alkyle ou d'aryle acrylate dans un solvant qui renferme au moins un monomère acrylique ;
> (ii) dispersion dans le mélange fluidique issu de l'étape (i) d'une quantité supplémentaire d'un polymère/copolymère d'alkyle ou d'aryle acrylate ; et
> (iii) durcissement du matériau résultant pour former une matière solide,

> dans lequel la quantité de polymère/copolymère d'alkyle ou d'aryle acrylate dispersée à l'étape (i) est inférieure à 20 % poids/volume, et dans lequel le polymère/copolymère d'alkyle ou d'aryle acrylate de l'étape (i) contient moins de 0,1 % poids/poids d'un initiateur de polymérisation à radical libre qui est un peroxyde ou un percarbonate.

2. Procédé selon la revendication 1, dans lequel le polymère/copolymère d'alkyle ou d'aryle acrylate de l'étape (i) contient moins de 0,08 % poids/poids d'un initiateur de polymérisation à radical libre.

3. Procédé selon la revendication 1 ou la revendication 2, dans lequel l'initiateur de polymérisation à radical libre est un peroxyde.

4. Procédé selon l'une quelconque des revendications précédentes, dans lequel l'initiateur de polymérisation à radical libre est le peroxyde de benzoyle.

5. Procédé selon la revendication 1 ou la revendication 2, dans lequel l'initiateur de polymérisation à radical libre est un percarbonate.

6. Procédé selon l'une quelconque des revendications précédentes, dans lequel le solvant est constitué d'au moins deux monomères acryliques.

7. Procédé selon l'une quelconque des revendications précédentes, dans lequel le polymère d'alkyle ou d'aryle acrylate de l'étape (i) et/ou de l'étape (ii) est le méthacrylate de polyméthyle.

8. Procédé selon l'une quelconque des revendications précédentes, dans lequel le polymère d'alkyle ou d'aryle acrylate de l'étape (i) et/ou de l'étape (ii) présente un poids moléculaire moyen d'au moins 100.000.

9. Procédé selon la revendication 8, dans lequel le poids moléculaire moyen est compris entre 500.000 et 2.000.000.

10. Procédé selon l'une quelconque des revendications précédentes, dans lequel au moins un monomère acrylique est le méthacrylate de méthyle.

11. Procédé selon l'une quelconque des revendications précédentes, dans lequel le mélange fluidique formé à l'étape (i) présente une viscosité comprise entre 500 et 1800 cP.

12. Procédé selon l'une quelconque des revendications précédentes, dans lequel la matière issue de l'étape (ii) présente une viscosité supérieure à celle du mélange fluidique issu de l'étape (i).

13. Procédé selon l'une quelconque des revendications précédentes, dans lequel le durcissement est réalisé à une température d'au moins 50° C pendant moins de 2 heures.

14. Procédé selon la revendication 13, dans lequel le durcissement est réalisé à une pression d'au moins 60 psig.

15. Procédé selon l'une quelconque des revendications précédentes, dans lequel la matière formée à l'étape (ii) est insérée dans un moule avant le durcissement.

16. Procédé selon la revendication 15, dans lequel le moule est un porte-empreinte dentaire.